# FASCICULE DE BREVET EUROPEEN

(11) **EP 1 015 618 B1**
(45) Date de publication et mention de la délivrance du brevet: **02.07.2003**
(21) Numéro de dépôt: 98920613.1
(22) Date de dépôt: 10.04.1998
(51) Int. Cl.: C12N 15/86, C07K 14/72, C12N 7/02

(54) **PREPARATION DE RECEPTEURS MEMBRANAIRES A PARTIR DE BACULOVIRUS EXTRACELLULAIRES**
HERSTELLUNG VON MEMBRANREZEPTOREN MIT EXTRAZELLULÄREN BACULOVIRUS
PREPARING MEMBRANE RECEPTORS FROM EXTRACELLULAR BACILOVIRUSES

(30) Priorité: 11.04.1997 FR 9704476
(43) Date de publication de la demande: 05.07.2000
(73) Titulaire: CENTRE NATIONAL DE LA RECHERCHE SCIENTIFIQUE (CNRS), 75794 Paris Cedex 16 (FR); UNIVERSITE DE MONTREAL, Montréal, Québec H3T 1J4 (CA)
(72) Inventeur: BOUVIER, Michel, Montreal, Quebec H3T 1B7 (CA); LOISEL, Thomas, Montreal, Quebec H3T 1J7 (CA); MARULLO, Stefano, F-75013 Paris (FR); BOULANGER, Pierre, F-34000 Montpellier (FR); STROSBERG, Arthur, Donny, F-75015 Paris (FR)
(74) Mandataire: Vialle-Presles, Marie José
(86) Numéro de dépôt international: FR9800736
(87) Numéro de publication internationale: WO98046777

(56) Documents cités:
- WO-A-88/07082
- WO-A-96/09074
- WO-A-96/38575
- RAIVIO E ET AL: "EXPRESSION OF THE HUMAN INTERLEUKIN-2 RECEPTOR-GAMMA CHAIN IN INSECT CELLS USING A BACULOVIRUS EXPRESSION VECTOR" SCANDINAVIAN JOURNAL OF IMMUNOLOGY, 1995, 41, 338-342, XP002051485
- LOISEL T P ET AL: "Recovery of homogeneous and functional beta-2-adrenergic receptors from extracellular baculovirus particles" NATURE BIOTECHNOLOGY, 15 (12). 1997. 1300-1304., XP002051486

## Description

L'invention est relative à la production de récepteurs membranaires dans un système baculovirus/cellules d'insecte.

Dans les dernières années, des systèmes d'expression hétérologues ont souvent été utilisés pour étudier l'expression, ainsi que les caractéristiques pharmacologiques et biochimiques des récepteurs membranaires.

Bien qu'une expression significative puisse être obtenue dans certains systèmes d'expression en cellules de mammifère, des problèmes se sont posés, en particulier dans le cas de certains types de récepteurs tels que les récepteurs couplés aux protéines G.

Les récepteurs couplés aux protéines G appartiennent à la super-famille des récepteurs à sept domaines transmembranaires. Ils comprennent, par exemple, les récepteurs adrénergiques ou muscariniques, et ont tous la même structure qui est faite d'une chaîne polypeptidiques comprenant sept domaines hydrophobes qui traversent la double couche lipidique membranaire.

Lorsque l'on cherche à exprimer ces récepteurs dans des systèmes de cellules de mammifère, on obtient généralement une densité relativement faible de récepteurs exprimés par lesdites cellules, excédant rarement quelques picomoles de récepteur par milligramme de protéine membranaire. Bien que ces niveaux d'expression soient suffisants pour une caractérisation fonctionnelle et pharmacologique, ils limitent clairement le type d'études biochimiques, biophysiques et structurelles qui peuvent être effectuées. A fortiori, ce système d'expression ne peut pas être utilisé pour la production de récepteurs en grande quantité, par exemple pour leur utilisation thérapeutique.

Afin d'augmenter la quantité de récepteurs obtenus, différentes équipes ont cherché à les produire dans un système baculovirus/cellule d'insecte : dans de nombreux cas, des baculovirus exprimant des récepteurs couplés aux protéines G ont pu produire ces récepteurs recombinants dans des cellules des lignées Sf9 ou Sf21 de *Spodoptera frugiperda*, jusqu'à des niveaux atteignant 30 à 100 picomoles par milligramme de protéine membranaire. Ces systèmes ont permis de faire des progrès significatifs dans l'étude de la palmitoylation des récepteurs et également d'étudier les effets produits par différents agonistes et antagonistes, ou bien de procéder à la reconstitution de récepteurs artificiels.

Cependant, le système baculovirus/cellules d'insecte présente l'inconvénient majeur d'exprimer une importante proportion de récepteurs inactifs. Les récepteurs, qui sont récupérés dans la fraction membranaire des cellules infectées par les baculovirus sont sous forme immature et incomplètement glycosylée. Ceci résulte probablement d'une saturation de la voie normale de maturation post-traductionnelle, qui entraîne la rétention de récepteurs immatures dans les membranes du réticulum endoplasmique ou dans l'appareil de Golgi. Pour obtenir des récepteurs fonctionnels on est dans ce cas obligé d'inclure une étape de purification basée sur l'activité biologique du récepteur, (par exemple, une étape de chromatographie d'affinité).

Il serait donc nécessaire de mettre au point un système permettant de séparer facilement la membrane plasmique comprenant les récepteurs matures, des autres fractions membranaires tel que le réticulum endoplasmique ou les membranes de l'appareil de Golgi, qui comprennent le récepteur immature, biologiquement inactif.

Il a été récemment montré que l'infection de cellules Sf9 par un baculovirus codant pour le gène Gag de HIV1 (Pr55 Gag) entraîne le bourgeonnement de particules portant la protéine Gag (particules Gag) qui sont relarguées dans le milieu extracellulaire. Il a été suggéré que ces particules Gag entraînaient lors de leur bourgeonnement, la membrane plasmique et les protéines qui lui sont associées.

Les Inventeurs ont formulé l'hypothèse que la co-expression dans un système baculovirus/cellules d'insectes, d'un récepteur couplé aux protéines G et de Pr55 Gag peut favoriser le relargage des particules Gag exprimant uniquement des récepteurs matures correctement insérés dans la membrane plasmique. Pour tester cette hypothèse, les Inventeurs ont infecté des cellules Sf9 avec des baculovirus codant le récepteur adrénergique humain β2AR et la protéine Pr55 Gag. De façon surprenante, ils ont alors constaté que le récepteur β2AR est presque totalement absent des particules Gag, mais est en revanche présent à forte densité dans des particules de baculovirus extracellulaires. En outre, les récepteurs exprimés dans ces baculovirus extracellulaires sont correctement glycosylés et normalement actifs.

La présente invention a concerne l'utilisation de ces baculovirus extracellulaires pour l'obtention de préparations d'un récepteur membranaire.

La présente invention a pour objet un procédé de production d'un récepteur membranaire recombinant dans un système baculovirus/cellule d'insecte, à partir d'une culture de cellules d'insectes infectées par un baculovirus recombinant exprimant le gène codant pour ledit récepteur membranaire, lequel procédé est caractérisé en ce que l'on obtient ledit récepteur membranaire à partir des baculovirus extracellulaires produits par lesdites cellules infectées.

Selon un mode de mise en oeuvre préféré de la présente invention, ledit récepteur appartient à la super-famille des récepteurs à sept domaines transmembranaires ; il s'agit par exemple d'un récepteur de la famille des récepteurs couplés aux protéines G.

Des baculovirus recombinants exprimant le gène codant pour le récepteur membranaire que l'on souhaite produire sont obtenus par clonage dudit gène sous contrôle transcriptionnel d'un promoteur approprié dudit baculovirus, selon des méthodes bien connues en elles-mêmes de l'homme de l'art.

N'importe quel promoteur fort de baculovirus utilisable pour l'expression de gènes hétérologues, tel par exemple que le promoteur de la polyédrine (*polh*) ou celui de la protéine P10, peut être employé pour l'obtention d'un baculovirus recombinant utilisable dans le cadre de la présente invention.

Selon un mode de mise en oeuvre préféré du procédé conforme à l'invention, il comprend une étape au cours de laquelle on procède à la récolte des baculovirus extracellulaires produits par lesdites cellules infectées et à leur séparation des fractions cellulaires. La récolte et la séparation des baculovirus extracellulaires peuvent être effectuées par centrifugations successives, par exemple de la manière suivante : on effectue une première centrifugation à environ 500xg, à l'issue de laquelle on récupère le surnageant contenant les baculovirus extracellulaires. Ce surnageant est soumis à une centrifugation à environ 45000×g ; le culot résultant qui contient les baculovirus extracellulaires est remis en suspension, et la suspension est soumise à une centrifugation à environ 500×g ; le surnageant résultant de cette centrifugation est centrifugé à environ 45000×g, et l'on récupère le culot, qui contient les baculovirus extracellulaires. Avantageusement, les baculovirus extracellulaires peuvent également être purifiés par centrifugation sur gradient de saccharose, ou tout autre procédé équivalent.

Selon un autre mode de mise en oeuvre préféré du procédé conforme à l'invention, il comprend une étape au cours de laquelle on procède à la lyse des baculovirus extracellulaires produits par lesdites cellules infectées ; avantageusement, il comprend également une étape au cours de laquelle on procède au fractionnement du lysat obtenu à l'issue de l'étape précédente, et à la récupération de la fraction comprenant ledit récepteur membranaire.

Les préparations purifiées et les lysats de baculovirus extracellulaires, ainsi que leurs fractions comprenant le récepteur membranaire, susceptibles d'être obtenus par les procédés définis ci-dessus constituent des préparations de récepteur membranaire qui font également partie de l'objet de la présente invention. Ces préparations sont constituées par des récepteurs actifs et totalement matures, contrairement aux préparations de récepteurs membranaires obtenues dans l'art antérieur à partir des membranes plasmiques de cellules infectées, qui comprennent une forte proportion de récepteurs inactifs, et qui ne peuvent être utilisées qu'après une étape supplémentaire de purification sur la base de l'activité des récepteurs concernés, par exemple après chromatographie d'affinité.

Au contraire, les préparations de récepteurs membranaires conformes à l'invention sont caractérisées en ce que, préalablement à toute purification effectuée sur la base de l'activité du récepteur concerné, au moins 90%, et de préférence au moins 95% dudit récepteur est sous forme active.

Des préparations, conformes à l'invention, d'un récepteur membranaire peuvent être utilisées pour préparer ledit récepteur sous forme purifiée, avec un bien meilleur rendement que celui auquel on pouvait parvenir à partir des préparations de récepteurs membranaires obtenues dans l'art antérieur à partir des membranes plasmiques des cellules infectées.

Les préparations de récepteurs membranaires conformes à l'invention, ainsi que les baculovirus extracellulaires obtenus lors de la mise en oeuvre du procédé conforme à l'invention, peuvent également être utilisés directement, par exemple comme système d'étude des propriétés de récepteurs membranaires, comme système de criblage de molécules actives sur ces récepteurs membranaires, ou bien pour étudier leurs modifications post-traductionnelles telles que la phosphorylation ou la palmitoylation.

La présente invention sera mieux comprise à l'aide du complément de description qui va suivre, qui se réfère à des exemples de mise en oeuvre du procédé conforme à l'invention pour la préparation de récepteurs membranaires.

### EXEMPLE 1 : PREPARATION DE BACULOVIRUS RECOMBINANTS EXPRIMANT UN RECEPTEUR COUPLE AUX PROTEINES G

Un baculovirus recombinant exprimant β2AR est obtenu en clonant une séquence d'ADN constituée par l'ADNc de β2AR en fusion avec l'épitope *c-myc*, obtenue comme décrit par MOUILLAC et al. [J. Biol. Chem., 267, 21733-21737 (1992)], au site NheI du vecteur de transfection/recombinaison pJVNheI(commercialisé par la société INVITROGEN). Ce vecteur a été transfecté avec le génome linéarisé d'un baculovirus AcMNPV (commercialisé par la société INVITROGEN) dans des cellules Sf9 ; le baculovirus recombinant obtenu de la sorte est dénommé c-*myc*-β2AR.

De la même manière, on a cloné la séquence codant pour le récepteur muscarinique M1 et la séquence codant pour le récepteur dopaminergique D1 [respectivement décrites par ALLARD et al. Nucleic Acid Research, 15, p 10604, (1987) et par DEARRY et al., Nature, 347, p 72, (1990)] pour obtenir les baculovirus recombinants (respectivement dénommés M1-R et D1-R) exprimant ces récepteurs.

### a) Culture et infection des cellules, et récolte des baculovirus extracellulaires :

Des cellules Sf9 sont cultivées à 27°C dans des flacons de culture en suspension de 100 ml (BELLCO GLASS) en milieu de GRACE supplémenté (GIBCO) contenant 10% de sérum de veau foetal (FBS), et 0,001% d'acide pluronique. 60 ml de suspension de cellules (2 × 10⁶/ml) sont infectées avec le baculovirus recombinant exprimant β2AR, D1, ou M1, à une multiplicité d'infection variant entre 2 et 5.

Les cellules sont récoltées par centrifugation à 500 × g pendant 5 min. à 4°C.

Les particules virales sont isolées après récolte des cellules, par centrifugation du surnageant de culture à 45,000 × g pendant 20 min. à 4°C. Les culots obtenus sont resuspendus à 4°C dans un volume de tampon phosphate salin (PBS) égal à 1/10^{éme} du volume de la culture initiale, et centrifugés à 500 × g, pendant 5 minutes à 4°C ; le surnageant de cette centrifugation à 500 × g est à nouveau centrifugé à 45,000 × g pendant 20 mm à 4°C.

### b) Purification des particules de baculovirus sur gradient de saccharose

Le culot de particules virales obtenu à partir de 100 ml de cultures de cellules Sf9 infectées par le baculovirus recombinant exprimant β2AR, M1, ou D1, est resuspendu dans 1,2 ml de solution TE (10 mM Tris-HCl, 1 mM EDTA, pH 7,4), en présence d'inhibiteurs de protéases).

La suspension est déposée au sommet d'un tube contenant un gradient linéaire (25%-56%) de saccharose en solution TE. Les tubes sont centrifugés à 100,000 × g pendant 90 minutes. Le gradient est collecté du sommet au fond du tube, en 20 fractions. La première fraction a un volume de 1,4 ml, et les 19 autres sont de 500 µl.

### EXEMPLE 2 : MISE EN EVIDENCE DE L'ACTIVITE β2AR DANS LES BACULOVIRUS RECOMBINANTS EXTRACELLULAIRES

Les cellules Sf9 infectées par le baculovirus recombinant exprimant β2AR sont mises en culture, et les baculovirus sont récoltés comme décrit à l'exemple 1 a), 24 heures, 48 heures, 72 heures, 96 heures, et 120 heures après l'infection.

L'activité des récepteurs β2AR est évaluée par des mesures de saturation et de liaison compétitive effectuées comme décrit par BOUVIER et al. [Mol. Pharmacol. 33 :133-139 (1982)] en utilisant du [¹²⁵I]-iodocyanopindolol ([¹²⁵I]ICYP) comme ligand marqué.

Les culots de particules virales obtenus sont resuspendus à 4°C dans le tampon qui sera utilisé pour la réaction. Des aliquotes de cette supension de particules virales, correspondant à 0,2 à 1 µg de protéines sont mélangés avec 5 à 350 pM de radioligand [¹²⁵I]ICYP dans un volume final de 500 µl. La liaison non-spécifique est évaluée en utilisant 10 µM d'alprénolol.

Dans ces conditions, l'activité β2AR est détectée dans les surnageants des cultures cellulaires à partir de 48 heures après l'infection, atteint son maximum 72 heures après l'infection, et reste constante jusqu'à 120 heures après l'infection.

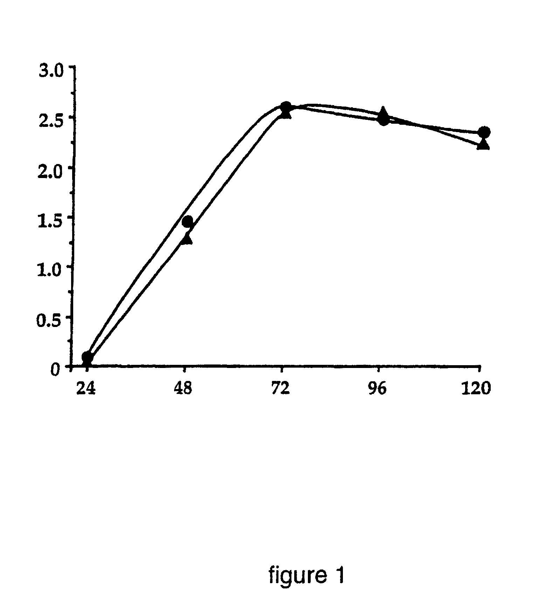

Ces résultats sont illustrés par la Figure 1.

Cette figure montre également les résultats obtenus, dans les mêmes conditions expérimentales, sur des surnageants de cultures de cellules infectées à la fois avec le baculovirus recombinant *c-myc*-β2AR, et un baculovirus recombinant exprimant la protéine Gag de HIV (● = β2AR ; ▲ = β2AR + Gag). On constate que contrairement à ce qui était initialement attendu, la présence de la protéine Gag n'augmente pas la quantité de β2AR dans les surnageants de culture.

On constate également que l'activité β2AR détectée dans les surnageants ne provient pas de la lyse cellulaire, dans la mesure où cette activité apparaît 48 heures après l'infection, c'est à dire à un moment où la majorité des cellules infectées sont encore viables, et n'augmente pas entre 72 et 120 heures après l'infection, malgré la lyse cellulaire importante qui se produit à ce moment là.

La nature des particules du surnageant portant l'activité β2AR a été vérifiée par microscopie électronique, après marquage de ces particules à l'aide d'un anticorps dirigé contre l'antigène c-myc, ou d'un anticorps dirigé contre le récepteur β2AR. Il a ainsi été constaté que les particules reconnues par l'un ou l'autre de ces anticorps sont des bâtonnets de 15 × 100 nm, ce qui correspond à des baculovirus extracellulaires.

Dans le cas de la co-infection avec un baculovirus exprimant la protéine Gag, on observe en outre dans le surnageant la présence de particules présentant la morphologie des particules Gag, et reconnues par un anticorps anti-Gag ; cependant, contrairement aux baculovirus extracellulaires, ces particules Gag ne sont que très faiblement reconnues par les anticorps anti-c-myc et anti-β2AR.

La présence du récepteur β2AR a également été vérifiée dans les préparations de baculovirus recombinant *c-myc*-β2AR purifiées sur gradient de saccharose, comme décrit à l'exemple 1 b) ci-dessus..

L'activité β2AR a été déterminée selon le protocole décrit à l'Exemple 2 ci dessus, sur les différentes fractions du gradient.

Parallèlement, la détection des antigènes vp80, gp67, et vp39 du baculovirus AcMNPV, en utilisant un anticorps polyclonal dirigé contre ces antigènes, a été effectuée sur les mêmes fractions. Les résultats obtenus montrent que l'activité β2AR co-sédimente avec les particules virales.

L'ensemble des résultats obtenus ci-dessus montre que non seulement des molécules du récepteur sont exprimées dans les baculovirus extracellulaires recombinants, mais encore qu'il s'agit de molécules actives.

La quantification de l'activité β2AR dans les préparations de baculovirus extracellulaires recombinants purifiées sur gradient de saccharose permet d'évaluer la densité du récepteur actif à environ 25 pmol/mg de protéines totales.

### EXEMPLE 3 : COMPARAISON DES FORMES DU RECEPTEUR β2AR PRESENTES DANS DES PREPARATIONS DE MEMBRANES CELLULAIRES ET DANS LES BACULOVIRUS EXTRACELLULAIRES

Des cellules Sf9 infectées avec le baculovirus recombinant *c-myc-*β2AR sont récoltées 72 heures après l'infection. Les baculovirus extracellulaires *c-myc*-β2AR sont récoltés à partir du surnageant de culture de ces cellules, et les particules virales sont purifiées comme décrit à l'exemple 1 b).

Les membranes des cellules Sf9 sont préparées comme suit : les cellules sont centrifugées à 500 × g pendant 5 minutes à 4°C, rincées une fois avec du tampon PBS à 4°C, et resuspendues dans du tampon de lyse (20 mM Tris-HCl, 5 mM EDTA, pH7,4 contenant 5 µg/ml leupeptine, 5 µg/ml d'inhibiteur de trypsine et 10 µg/ml de benzamidine) à 4°C. Les cellules sont alors lysées par sonication, les lysats sont centrifugés 5 min à 500×g à 4°C et les surnageants centrifugés à 45,000×g pendant 20 mm à 4°C. Les culots sont resuspendus à 4°C dans du tampon de réaction (75 mM Tris-HCl (pH 7,4), 12,5 mM chlorure de magnésium, 2 mM EDTA), en présence d'inhibiteurs de protéases.

6 mg de la préparation de membranes cellulaires, ou bien de la préparation de baculovirus purifiés sont ajoutés à 5 ml de tampon de solubilisation (10 mM Tris-HCl, 100 mM NaCl, 2 mM EDTA, pH 7,4, 0,3% n-dodécyl maltoside (BOEHRINGER MANNHEIM) en présence d'inhibiteurs de protéases. La solubilisation est effectuée pendant 90 min à 4°C.

Les récepteurs solubilisés sont purifiés par chromatographie d'affinité comme décrit ci-dessous. La matrice d'affinité ALPRENOLOL-SEPHAROSE est synthétisée selon la méthode de BENOVIC et al.[J. Biol. Chem., 262 :9026-9032, (1987)]. Cette matrice est utilisée pour purifier le *c-myc*-β2AR selon le protocole décrit par MOUILLAC et al. [J. Biol. Chem., 267 :21733-21737, (1992)]. Tous les tampons comprennent du n-dodécyl maltoside (0,05%).

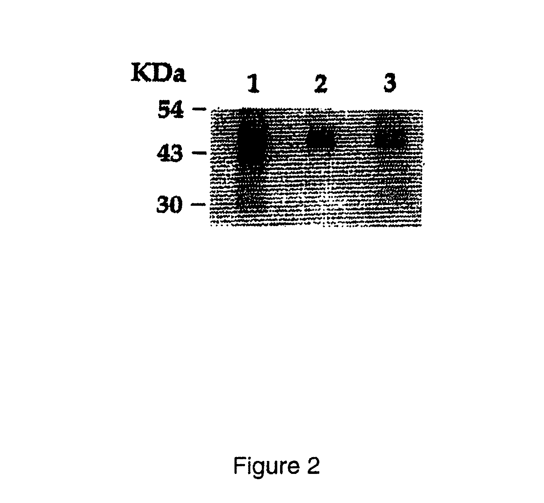

Les préparations obtenues après chromatographie d'affinité sont concentrées en utilisant des cartouches CENTRIPREP et CENTRICON (AMICON) et la quantité de *c-myc-*β2AR dans chaque échantillon est déterminée en utilisant du [¹²⁵I]-iodocyanopindolol ([¹²⁵I]ICYP) comme décrit par MOUILLAC et al. [J. Biol. Chem., 267 :21733-21737, (1992)].Les préparations de particules virales, de membranes, ou de β2AR purifiée par chromatographie d'affinité sont soumises à une électrophorèse sur gel de polyacrylamide en présence de SDS (SDS-PAGE), en conditions non-réductrices, sur des plaques de gel à 10%. Les protéines séparées sur les gels sont transférées sur nitrocellulose et révélées avec un anticorps monoclonal de souris anti-c-rnyc, et un second anticorps anti-souris couplé à la phosphatase alcaline ou à la peroxydase de raifort. Les résultats sont illustrés par la Figure 2.

Le transfert de Western de la préparation de membranes cellulaires (Figure 2, piste 1) montre la présence de plusieurs bandes immunoréactives, entre 40 et 50 kDa.

Le transfert de Western des préparations de β2AR purifiée par chromatographie d'affinité (Figure 2, piste 2) montre une seule et large bande immunoréactive, entre 46 et 50 kDa, qui représente la forme mature, biologiquement active, du récepteur β2AR.

Le transfert de Western de la préparation de baculovirus extracellulaires purifiés (Figure 2, piste 3) montre également la présence d'une seule et large bande immunoréactive entre 46 et 50 kDa.

Ces résultats montrent que les molécules de récepteur β2AR présentes dans les baculovirus extracellulaires représentent uniquement la forme biologiquement active, contrairement aux molécules de récepteur β2AR présentes dans les préparations de membranes cellulaires, qui représentent un mélange de formes actives et inactives.

### EXEMPLE 4 : PROPRIETES PHARMACOLOGIQUES DE DIFFERENTS RECEPTEURS EXPRIMES DANS LES BACULOVIRUS EXTRACELLULAIRES.

Des préparations de baculovirus extra-cellulaires exprimant les récepteurs β2AR, M1, ou D1 sont obtenues comme décrit dans l'exemple 1 ci-dessus.

La liaison de chacun des récepteurs au ligand est évaluée comme décrit dans l'exemple 2 ci-dessus.

Les essais de liaison compétitive en présence d'agonistes sont effectuées en utilisant 70 pM de [¹²⁵I]ICYP comme radioligand. La concentration du ligand non-marqué varie de 10⁻⁴ à 10⁻¹² M.

Les dosages de saturation des récepteurs M1-muscariniques (M1-R) et D1-dopaminergiques (D1-R) exprimés dans les particules virales sont effectuées en utilisant 1-100 nM [3H]-pirenzepine (NEN, DUPONT) et 0,02-3 nM [¹²⁵I]-R(+)SCH-23390 (NEN, Dupont) avec 5-10 µg ou 1-2 µg de protéines pour M1-R et D1-R respectivement. Pour évaluer la liaison non-spécifique, on ajoute au mélange réactionnel 1 µM Atropine (RBI) pour M1-R, et 10 µM haloperidol (RBI) pour D1-R.

Les résultats de ces expérimentations sont illustrés par le tableau I ci-après.

**TABLEAU I**

| Récepteur | Ligand | Kd pM | BMax pmol/mg de protéine | Ki µM |
|---|---|---|---|---|
| β2AR | [¹²⁵I]ICYP | 49,4 ± 11,5 | | |
| | Epinephrine | | | 8,98 ± 4,02 |
| | Arterenol | | | 3,27 ± 0,38 |
| M1 | [³H]-Pirenzepine | 1360 ± 670 | 5,56 ± 0,46 | |
| D1 | [¹²⁵I]-SCH23390 | 118 ± 63 | 5,21 ± 0,84 | |

Ces résultats montrent que différents récepteurs de la famille des récepteurs couplés aux protéines G sont exprimés sous forme active dans des baculovirus extra-cellulaires.

### EXEMPLE 5 : PALMITOYLATION DU RECEPTEUR β2AR EXPRIME DANS DES BACULOVIRUS EXTRA-CELLULAIRES.

Les particules virales exprimant *c-myc*-β2AR, sont préparées comme décrit à l'exemple 1 ci-dessus, et le culot resuspendu dans du PBS. 1 mCi de [³H]palmitate dissous dans du diméthyl sulfoxyde est ajouté aux particules virales. La réaction est effectuée pendant des durées déterminées en présence ou bien en absence de 1 µM (concentration finale) d'isoprotérénol.

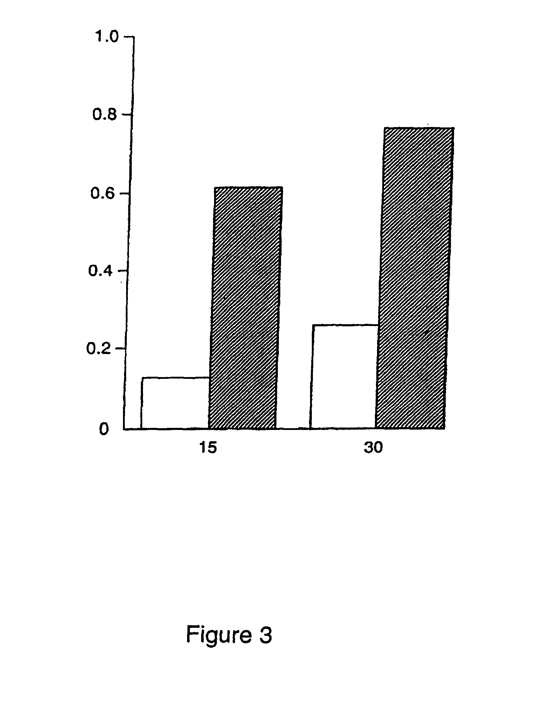

Les résultats sont illustrés par la Figure 3 : Légende de la figure 3 :
□ : incorporation en l'absence d'isoprotérénol ;
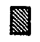 : incorporation en présence d'isoprotérénol).

### EXEMPLE 6 : COMPARAISON DES FORMES DU RECEPTEUR β2AR

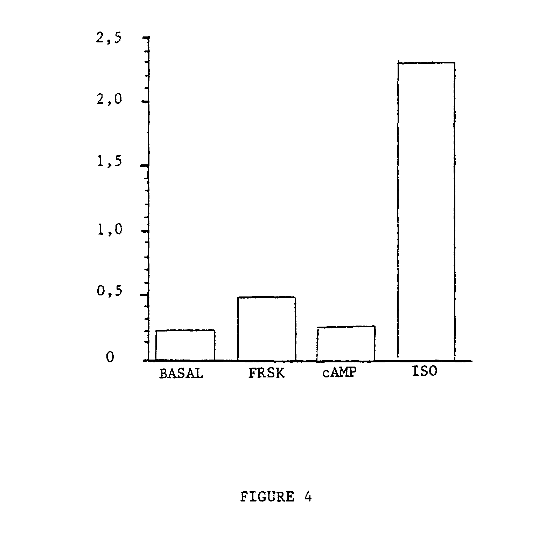

Les particules virales exprimant *c-myc*-β2AR sont préparées comme décrit à l'exemple 1 ci-dessus, et le culot resuspendu dans un tampon (100 mM Tris-HCl, 10 mM MgCl₂, pH 7,4 et des inhibiteurs de protéases). On mélange 1 volume de baculovirus extracellulaires et 1 volume de mélange de phosphorylation (2,3 µCi/µl de [γ³²P]ATP, 10 mM Tris-HCl, 2 mM MgCl₂ pH 7,4, 25 mM phosphoénol pyruvate, 0,3 mM GTP, 1 mM ATP, 4 U/ml de pyruvate kinase, et 20 U/ml de myokinase). La réaction est effectuée pendant 25 min. à 30°C. A la fin de la réaction, l'incorporation de ³²P est mesurée en l'absence d'activateur (témoin) ou en présence de 1 µM d'isoprotérénol, ou de 100 µM de dibutyril AMP cyclique, ou de 100 µM de forskoline. Les résultats sont illustrés par la figure 4.
Légende de la figure 4 :
En ordonnée : incorporation relative de ³²P (unités arbitraires)
En abscisse :
   BASAL : témoin
   FRSK : incorporation en présence de forskoline
   cAMP : incorporation en présence de dibutyril AMP cyclique
   ISO : incorporation en présence d'isoprotérénol

### EXEMPLE 7 : FONCTIONNALITE DU RECEPTEUR β2AR EXPRIME DANS DES BACULOVIRUS EXTRA-CELLULAIRES

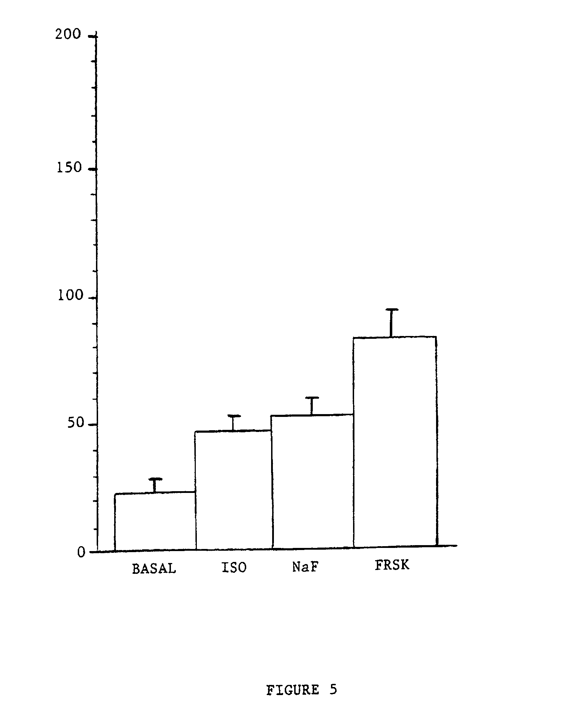

Les particules virales exprimant *c-myc*-β2AR sont préparées comme décrit à l'exemple 1 ci-dessus, et le culot resuspendu dans un tampon (75 mM Tris-HCl, 12,5 mM MgCl₂, 2 mM EDTA, pH 7,4 et des inhibiteurs de protéases). 20 µl de suspension de baculovirus extracellulaires sont mélangés à 30 µl de milieu réactionnel contenant de 0,2 mM ATP, 0,090 mM GTP, 0,20 mM cAMP, 0,20 mM isobutylméthylxanthine, 1 µCi [γ³²P]ATP, 5 mM phosphoénolpyruvate, 0,3 U de pyruvate kinase, et 2 U de myokinase. Après 30 min. d'incubation à 37°C, les réactions sont arrétées par l'ajout de 1 ml de solution d'arrêt (0,4 mM ATP, 0,3 mM AMP cyclique, et 25000 cpm d'AMP cyclique tritié). L'activité a été déterminée en l'absence d'activateur (témoin) ou en présence de l'un des activateurs suivants : 1 µM d'isoprotérénol, 10 µM NaF, ou de 100 µM de forskoline. Les résultats sont exprimés en picomoles d'AMP cyclique produit par minute et par milligramme de protéine. Ces résultats sont illustrés par la Figure 5.
Légende de la figure 5 :
En ordonnée : activité adényl-cyclase (en picomoles d'AMP cyclique/min./mg de protéine)
En abscisse :
   BASAL : témoin
   FRSK : incorporation en présence de forskoline
   NaF : incorporation en présence de NaF
   ISO : incorporation en présence d'isoprotérénol

Ces résultats montrent que le récepteur β2AR présent dans les baculovirus extra-cellulaires est dans un environnement qui reproduit l'environnement membranaire naturel, et que les préparations de baculovirus extracellulaires peuvent donc être utilisées dans toutes les applications des récepteurs membranaires où une reproduction de cet environnement est souhaitable.

## Revendications

1. Procédé de production d'un récepteur membranaire recombinant dans un système baculovirus/cellules d'insecte, à partir d'une culture de cellules d'insectes infectées par un baculovirus recombinant exprimant le gène codant pour ledit récepteur membranaire, lequel procédé est **caractérisé en ce que** l'on obtient ledit récepteur membranaire à partir des baculovirus extracellulaires produits par lesdites cellules infectées.

2. Procédé selon la revendication 1 **caractérisé en ce que** ledit récepteur appartient à la super-famille des récepteurs à sept domaines transmembranaires.

3. Procédé selon la revendication 2, **caractérisé en ce que** ledit récepteur appartient à la famille des récepteurs couplés aux protéines G.

4. Procédé selon une quelconque des revendications 1 à 3, **caractérisé en ce qu'**il comprend une étape au cours de laquelle on procède à la récolte des baculovirus extracellulaires produits par lesdites cellules infectées, et à leur séparation d'avec les fractions cellulaires.

5. Procédé selon une quelconque des revendications 1 à 4, **caractérisé en ce qu'**il comprend en outre une étape au cours de laquelle on procède à la lyse desdits baculovirus extracellulaires.

6. Procédé selon la revendication 5, **caractérisé en ce qu'**il comprend également une étape au cours de laquelle on procède au fractionnement du lysat obtenu à l'issue de l'étape précédente, et à la récupération de la fraction comprenant ledit récepteur membranaire.

7. Préparation de récepteur membranaire, constituée par un lysat de baculovirus extracellulaires susceptible d'être obtenu par un procédé selon la revendication 5.

8. Préparation de récepteur membranaire selon la revendication 7, **caractérisée en ce que** préalablement à toute purification effectuée sur la base de l'activité du récepteur concerné, au moins 90%, et de préférence au moins 95% dudit récepteur est sous forme active.

9. Utilisation d'un baculovirus extracellulaire, produit par une culture de cellules d'insectes infectées par un baculovirus recombinant exprimant le gène codant pour un récepteur membranaire, pour l'obtention de préparations dudit récepteur membranaire.

10. Utilisation d'un baculovirus extracellulaire produit par une culture de cellules d'insectes infectées par un baculovirus recombinant exprimant le gène codant pour un récepteur membranaire, ou d'une préparation de récepteur membranaire selon une quelconque des revendications 7 ou 8, pour l'obtention d'un modèle d'étude des propriétés dudit récepteur membranaire.

11. Utilisation selon la revendication 10, **caractérisée en ce que** ledit baculovirus extracellulaire ou ladite préparation de récepteur membranaire sont utilisés pour le criblage de molécules actives sur ledit récepteur membranaire.

12. Utilisation selon la revendication 10, **caractérisée en ce que** ledit baculovirus extracellulaire ou ladite préparation de récepteur membranaire sont utilisés pour l'étude de modifications post-traductionnelles dudit récepteur membranaire.

## Patentansprüche

1. Verfahren zur Herstellung eines rekombinanten Membranrezeptors in einem System Baculovirus/Insektenzellen ausgehend von einer Kultur von Insektenzellen, die mit einem rekombinanten Baculovirus infiziert sind, das das Gen, das für den Membranrezeptor codiert, exprimiert, wobei das Verfahren **dadurch gekennzeichnet ist, dass** man den Membranrezeptor ausgehend von extracellulären Baculoviren, die von den infizierten Zellen produziert werden, erhält.

2. Verfahren nach Anspruch 1, **dadurch gekennzeichnet, dass** der Rezeptor zur Superfamilie der Sieben-Transmembrandomänen-Rezeptoren gehört.

3. Verfahren nach Anspruch 2, **dadurch gekennzeichnet, dass** der Rezeptor zur Familie der Rezeptoren gehört, die an G-Proteine gebunden sind.

4. Verfahren nach einem der Ansprüche 1 bis 3, **dadurch gekennzeichnet, dass** es eine Stufe umfasst, im Verlauf der man die Ernte der extracellulären Baculoviren, die von den infizierten Zellen produziert werden, und ihr Abtrennen von cellulären Fraktionen durchführt.

5. Verfahren nach einem der Ansprüche 1 bis 4, **dadurch gekennzeichnet, dass** es außerdem eine Stufe umfasst, im Verlauf der man die Lyse der extracellulären Baculoviren durchführt.

6. Verfahren nach Anspruch 5, **dadurch gekennzeichnet, dass** es auch eine Stufe umfasst, im Verlauf der man die Fraktionierung des in der vorangehenden Stufe erhaltenen Lysats und die Isolierung der Fraktion, die den Membranrezeptor umfasst, durchführt.

7. Präparation eines Membranrezeptors, die aus einem Lysat extracellulärer Baculoviren besteht, das nach einem Verfahren nach Anspruch 5 erhalten werden kann.

8. Präparation eines Membranrezeptors nach Anspruch 7, **dadurch gekennzeichnet, dass** vor jeder Reinigung, die auf der Basis der Aktivität des betreffenden Rezeptors durchgeführt wird, mindestens 90%, vorzugsweise mindestens 95% des Rezeptors in aktiver Form vorliegen.

9. Verwendung eines extracellulären Baculovirus, das durch eine Kultur von Insektenzellen produziert wird, die mit einem rekombinanten Baculovirus infiziert sind, das das Gen, das für den Membranrezeptor codiert, exprimiert, um Präparationen des Membranrezeptors zu erhalten.

10. Verwendung eines extracellulären Baculovirus, das durch eine Kultur von Insektenzellen produziert wird, die mit einem rekombinanten Baculovirus infiziert sind, das das Gen, das für den Membranrezeptor codiert, exprimiert, oder einer Präparation des Membranrezeptors nach Anspruch 7 oder 8, um ein Modell zur Untersuchung der Eigenschaften des Membranrezeptors zu erhalten.

11. Verwendung nach Anspruch 10, **dadurch gekennzeichnet, dass** das extracelluläre Baculovirus oder die Präparation des Membranrezeptors für die Durchmusterung von Molekülen, die gegenüber dem Membranrezeptor aktiv sind, verwendet werden.

12. Verwendung nach Anspruch 10, **dadurch gekennzeichnet, dass** das extracelluläre Baculovirus oder die Membranrezeptor-Präparation für die Untersuchung von post-translationalen Modifikationen des Membranrezeptors verwendet wird.

## Claims

1. Method of producing a recombinant membrane receptor in a baculovirus/insect cell system, from a culture of insect cells infected with a recombinant baculovirus expressing the gene encoding the said membrane receptor, which method is **characterized in that** the said membrane receptor is obtained from extracellular baculoviruses produced by the said infected cells.

2. Method according to Claim 1, **characterized in that** the said receptor belongs to the superfamily of receptors with seven transmembrane domains.

3. Method according to Claim 2, **characterized in that** the said receptor belongs to the family of G-protein-coupled receptors.

4. Method according to any one of Claims 1 to 3, **characterized in that** it comprises a step during which the extracellular baculoviruses produced by the said infected cells are harvested and they are separated from the cellular fractions.

5. Method according to any one of Claims 1 to 4, **characterized in that** it comprises, in addition, a step during which the said extracellular baculoviruses are lysed.

6. Method according to Claim 5, **characterized in that** it also comprises a step during which the lysate obtained at the end of the preceding step is fractionated, and the fraction comprising the said membrane receptor is recovered.

7. Preparation of membrane receptor constituted by an extracellular baculoviruses lysate which can be obtained by a method according to Claim 5.

8. Preparation of membrane receptor according to Claim 7, **characterized in that** prior to any purification carried out on the basis of the activity of the relevant receptor, at least 90%, and preferably at least 95%, of the said receptor is in an active form.

9. Use of an extracellular baculovirus, obtained from a culture of insect cells infected with a recombinant baculovirus expressing the gene encoding a membrane receptor, for the production of preparations of the said membrane receptor.

10. Use of an extracellular baculovirus, obtained from a culture of insect cells infected with a recombinant baculovirus expressing the gene encoding a membrane receptor, or of a preparation of membrane receptor as defined in either of Claims 7 and 8, for the production of a model for studying the properties of the said membrane receptor.

11. Use according to Claim 10, **characterized in that** the said extracellular baculovirus or the said preparation of membrane receptor are used for the screening of molecules which are active on the said membrane receptor.

12. Use according to Claim 10, **characterized in that** the said extracellular baculovirus or the said preparation of membrane receptor are used for studying post-traductionnal modifications of the said membrane receptor.
